# EUROPEAN PATENT SPECIFICATION

(11) **EP 3 320 560 B1**
(45) Date of publication and mention of the grant of the patent: **27.11.2019**
(21) Application number: 16753973.3
(22) Date of filing: 07.07.2016
(51) Int. Cl.: H01L 27/146

(54) **OPTICAL SENSOR WITH NARROW ANGULAR RESPONSE**
OPTISCHER SENSOR MIT ENGER WINKELREAKTION
CAPTEUR OPTIQUE À RÉPONSE ANGULAIRE ÉTROITE

(30) Priority: 07.07.2015 IT UB20151963
(43) Date of publication of application: 16.05.2018
(73) Proprietor: LFoundry S.r.l., 67051 Avezzano (IT)
(72) Inventor: SPAZIANI, Fabio, 67051 Avezzano (IT); DEL MONTE, Andrea, 67051 Avezzano (IT); MARGUTTI, Giovanni, 67051 Avezzano (IT); DE AMICIS, Giovanni, 67051 Avezzano (IT)
(74) Representative: Bongiovanni, Simone
(86) International application number: PCT/IB2016/054083
(87) International publication number: WO 2017/006278

(56) References cited:
- WO-A1-2014/014411
- WO-A1-2014/129968
- US-A1- 2007 290 284
- US-A1- 2013 019 461
- US-A1- 2013 037 700

## Description

### TECHNICAL FIELD OF THE INVENTION

The present invention relates to the sector of optical sensors and, specifically, to an optical sensor with narrow angular response. In particular, the present invention finds advantageous, but non-limitative, application in measuring devices, such as wearable biometric monitoring devices.

### BACKGROUND ART

Recent advances in miniaturization and detection accuracy of optical sensors have allowed the use of such devices in many fields.

For example, US 2007/0290284 A1 discloses an incident light angle detector to be used to adjust or compensate for the alignment of components of an optical sensing arrangement used in a metrology instrument (e.g., an optical encoder system) or other optical system. In particular, said detector determines the direction of illumination incident on a photosensitive device. Multiple mask layers include holes which form an interlayer optical path through which radiation reaches a photodetector. The interlayer optical path provides a selected nominal maximum signal angle and the detector senses when radiation is received at or near that angle.

Moreover, optical sensors are currently used also in wearable devices to monitor, in a non-invasive fashion, biometric parameters, such as heart rate and blood oxygenation. In particular, these wearable devices, such as watches, arm bands and headsets, can include:
- a light source, such as a laser or one or more Light Emitting Diodes (LEDs), configured to emit light having wavelength in visible and/or infrared spectrum so as to illuminate a target volume of a user's body;
- a detecting module including one or more optical sensors or detectors and designed to detect light from the target volume of the user's body; and
- a processing module configured to determine one or more biometric parameters of the user, such as heart rate and/or blood oxygenation, based on output signals from the detecting module.

For example, US 2014/0276119 A1 discloses a wearable heart rate monitoring device, which includes: a motion sensor configured to provide output corresponding to motion by a user wearing the wearable heart rate monitoring device, a heartbeat waveform sensor, and control logic. The control logic includes instructions for: (a) collecting concurrent output data from the heartbeat waveform sensor and output data from the motion detecting sensor, wherein the output data from the heartbeat waveform sensor provides information about the user's heart rate and wherein the output data from the motion detecting sensor provides information about the user's periodic physical movements other than heartbeats; (b) determining a periodic component of the output data from the motion detecting sensor; (c) using the periodic component of the output data from the motion detecting sensor to remove a corresponding periodic component from the output data from the heartbeat waveform sensor; (d) determining the user's heart rate; and (e) presenting the user's heart rate.

In particular, the heartbeat waveform sensor is a photoplethysmographic sensor having one or more light sources (e.g., LEDs or laser-based sources), a photo detector positioned to receive light emitted by the light source(s) after interacting with the user's skin, and circuitry determining the user's heart rate from output of the photo detector.

As for wearable biometric monitoring devices (such as that one according to US 2014/0276119 A1), it is to be noted that, if the optical sensors do not adhere perfectly to the user's skin, ambient light can affect the detection carried out by the optical sensors. In fact, due to the non-perfect contact between the optical sensors and the user's skin, a gap is interposed therebetween, which allows side light to reach the detectors, thereby interfering with the light from the target volume of the user's body and thus affecting, in particular reducing, Signal-to-Noise Ratio (SNR).

US 2014/0276119 A1 suggests a potential solution to this technical problem, namely the use of light-transmissive structures (in particular, structures created through an In-Mould-Labeling (IML) film formed over the photo detector and the light source(s)), wherein said light-transmissive structures may include a mask consisting of an opaque material that limits the aperture of one, some, or all of the light source (s) and/or detector. In this way, the light-transmissive structures may selectively "define" a preferential volume of the user's body that light is emitted into and/or detected from.

### OBJECT AND SUMMARY OF THE INVENTION

A general object of the present invention is, thence, that of overcoming the aforesaid technical problem of wearable biometric monitoring devices due to side light interference.

Moreover, a specific object of the present invention is that of providing an optical sensor for wearable biometric monitoring devices, which optical sensor has a narrow angular response such that to minimize, when used in a wearable biometric monitoring device, side light interference.

These and other objects are achieved by the present invention in that it relates to an optical sensor, as defined in the appended claims.

In particular, the present invention relates to an optical sensor based on Complementary Metal-Oxide-Semiconductor (CMOS) technology and comprising:
- a semiconductor substrate;
- an array of photocells, each of which
   - includes a respective photodetector active area that is formed in the semiconductor substrate and is exposed on a given planar surface of said semiconductor substrate, and
   - is designed to provide a respective output electrical signal related to incident light impinging on the respective photodetector active area;
- a multilayer structure, that includes metal and dielectric layers and is formed on the given planar surface of the semiconductor substrate; and
- light shielding means, that are formed in or on the multilayer structure and are made of one or more materials reflecting and/or absorbing incident light impinging on said light shielding means;
wherein each photodetector active area is associated with a corresponding optical path extending through the light shielding means and directed towards said photodetector active area to allow incident light with incident direction falling within a given direction range to reach said photodetector active area.

The optical sensor according to the present invention is characterized in that:
- all the photocells are connected in parallel to provide an overall output electrical signal related to incident light impinging on all the photodetector active areas; and
- all the optical paths are parallel to a given direction thereby causing all the photodetector active areas to be reached by incident light with incident direction parallel to said given direction.

Preferably, the optical paths are perpendicular to the given planar surface of the semiconductor substrate.

Preferably, each optical path is defined by one or more respective coaxial apertures formed in the light shielding means and centered on a respective axis parallel to said given direction.

Preferably, the photodetector active areas have one and the same given planar size on the given planar surface of the semiconductor substrate; the optical paths have one and the same given height and one and the same given width, said given height and width being related to said given planar size; and said given planar size, height and width, and relative position of each photodetector active area with respect to the corresponding optical path:
- define one and the same given angular range for all the photodetector active areas; and
- cause the photodetector active areas to be reached only by incident light with incident direction
   - parallel to said given direction, or
   - defining, with respect to said given direction, an incidence angle falling within said given angular range.

### BRIEF DESCRIPTION OF THE DRAWINGS

For a better understanding of the present invention, preferred embodiments, which are intended purely by way of example and are not to be construed as limiting, will now be described with reference to the attached drawings (not to scale), where:
- Figure 1 schematically illustrates working principle of light shielding means according to a first preferred embodiment of the present invention;
- Figure 2 shows an example of photocell with integrated light shielding means according to the first preferred embodiment of the present invention;
- Figures 3-9 show examples of steps for manufacturing the photocell of Figure 2;
- Figure 10 shows an example of photocell with light shielding means according to a second preferred embodiment of the present invention; and
- Figure 11 shows a comparison between angular responses of, respectively, a conventional optical sensor and an optical sensor according to the present invention.

### DETAILED DESCRIPTION OF PREFRRED EMBODIMENTS OF THE INVENTION

The following discussion is presented to enable a person skilled in the art to make and use the invention. Various modifications to the embodiments will be readily apparent to those skilled in the art, without departing from the scope of the present invention as claimed. Thus, the present invention is not intended to be limited to the embodiments shown and described, but is to be accorded the widest scope consistent with the principles and features disclosed herein and defined in the appended claims.

The present invention stems from Applicant's idea of integrating, into an optical sensor based on CMOS technology, light shielding means designed to define an angular range around a given incident direction and to suppress incident light with incidence angle outside the defined angular range. Preferably, said optical sensor equipped with the light shielding means can be integrated into a System-on-a-chip (SoC) along with read-out circuitry, Analogue Signal Processor (ASP), Digital Signal Processor (DSP), Analogue-to-Digital Converter (ADC), etc..

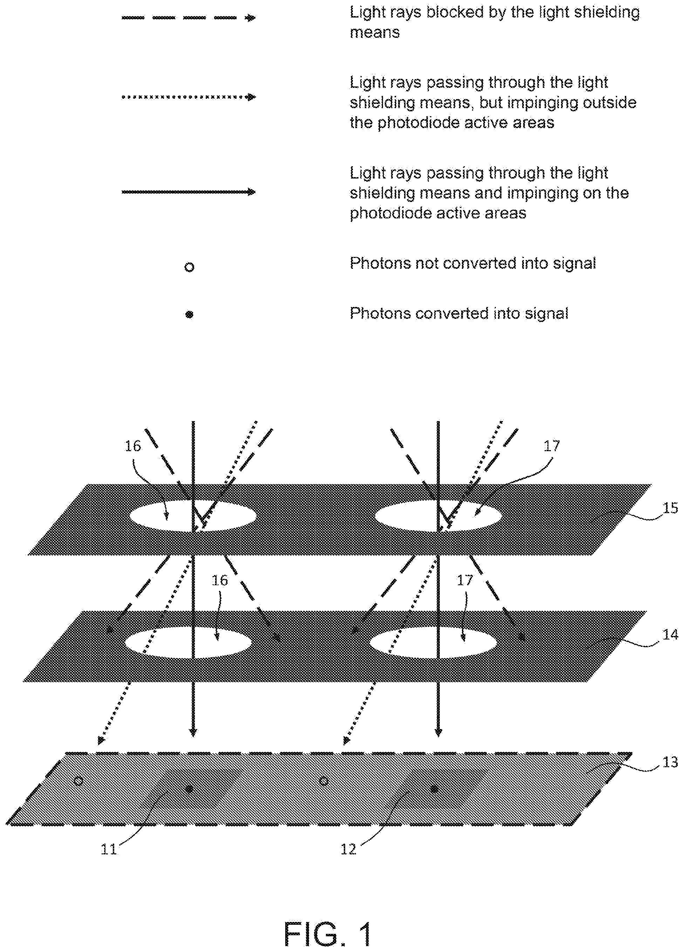

Figure 1 schematically illustrates working principle of light shielding means according to a first preferred embodiment of the present invention.

In particular, optical sensors realized in CMOS technology generally include single photosensitive units or photocells that, in turn, comprise respective photodetector active areas formed on a semiconductor substrate, as shown in Figure 1 where photodetector active areas 11 and 12 are sketched on a front side (planar) surface 13 of a semiconductor substrate (not shown).

Conveniently, the photodetector active areas 11 and 12 can be realized in the form of photodiode, phototransistor, or photoresistor active areas.

For example, CMOS-based photodiode active areas are realized, in the simplest form, as p-n (or n-p) junctions configured so that the n (or p) regions are depleted of charge carriers (such as electron/hole pairs) and, thence, incident photons generate electron/hole pairs collected by the depletion regions of the photodiodes. As is known, also pnp (or npn) junctions can be conveniently used (so-called "pinned photodiodes").

Therefore, the photons impinging on the photodetector active areas 11 and 12 are converted into charge carriers producing an output electric signal proportional to the intensity of the incident light, while photons impinging on the front side surface 13 of the semiconductor substrate, outside said photodetector active areas 11 and 12, are also converted into charge carriers, but are not collected by the photodetectors and, thence, do not contribute to the output electric signal.

The light shielding means according to said first preferred embodiment of the present invention include several metal layers configured, for each photocell, to:
- define an angular range with respect to a given incident direction with respect to said photocell;
- block incident light with incidence angle falling outside the defined angular range; and
- allow incident light with incidence angle falling within the defined angular range to propagate down to a respective photodetector active area.

In this respect, Figure 1 shows an example in which two metal layers 14 and 15, in the form of plates, are used as light shielding means. In particular, the metal plates 14 and 15 include first coaxial apertures 16 above the photodetector active area 11 and second coaxial apertures 17 above the photodetector active area 12 so that photons with incidence angle higher than a threshold defined by the aspect ratio of the series of apertures 16 and 17 are blocked (in particular are partially reflected and partially absorbed by the metal layers 14 and 15), while photons with incidence angle lower than said threshold reach the photodetector active areas 11 and 12 (except for those photons impinging on the front side surface 13 of the semiconductor substrate outside said photodetector active areas 11 and 12).

The apertures 16 and 17 can be circular, squared, rectangular or polygonal.

Conveniently, an antireflective coating can be deposited on the metallic layers 14 and 15 to minimize the reflection and maximize light absorption, thereby avoiding multiple reflections that can cause spurious rays directed toward the photodetector active areas 11 and 12.

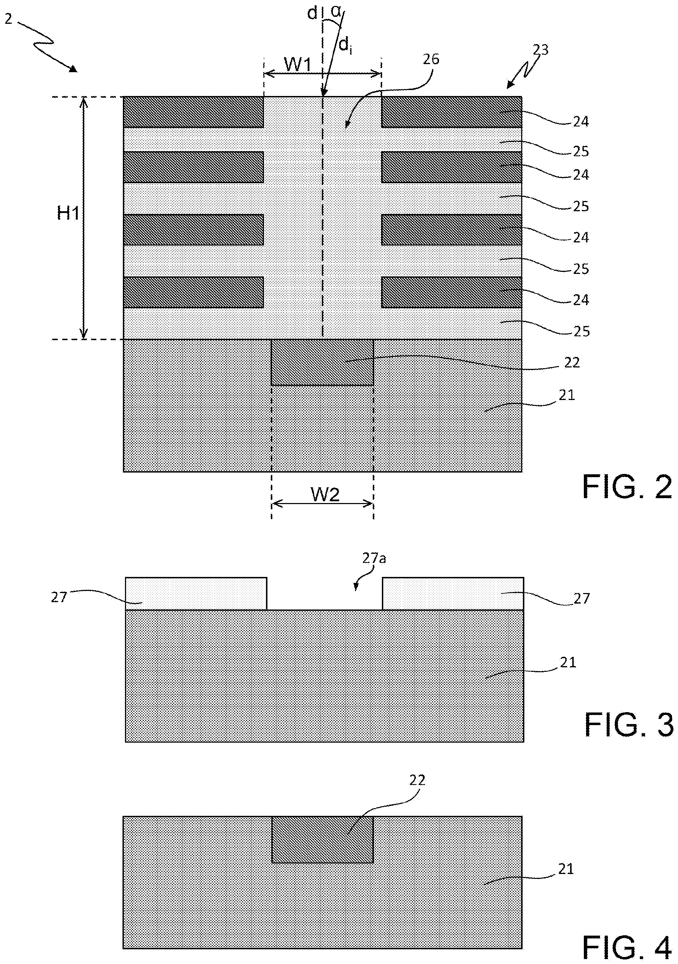

For a better understanding of the present invention, Figure 2 shows an example of photocell (denoted as a whole by 2) with integrated light shielding means according to said first preferred embodiment. In particular, Figure 2 is a cross-sectional view of the photocell 2, which includes:
- a semiconductor substrate 21 comprising a photodetector active area 22;
- a multilayer structure 23, formed on the semiconductor substrate 21 and comprising four metal layers 24 separated by four inter-level dielectric (ILD) insulating layers 25; and
- an optical path 26 formed through the metal layers 24 above the photodetector active area 22 (in particular defined by coaxial apertures formed in the metal layers 24 and centered on an axis d that passes through the centre of the photodetector active area 22).

Figures 3-9 show examples of steps for manufacturing the photocell 2.

In particular, Figure 3 shows a photoresist mask 27 formed on the semiconductor substrate 21 and patterned by a photoligraphic process to expose (through a window 27a) a portion of said semiconductor substrate 21 to one or more subsequent ion implantation processes intended to realize the photodetector active area 22.

Figure 4 shows the semiconductor substrate 21 on which the photodetector active area 22 has been realized.

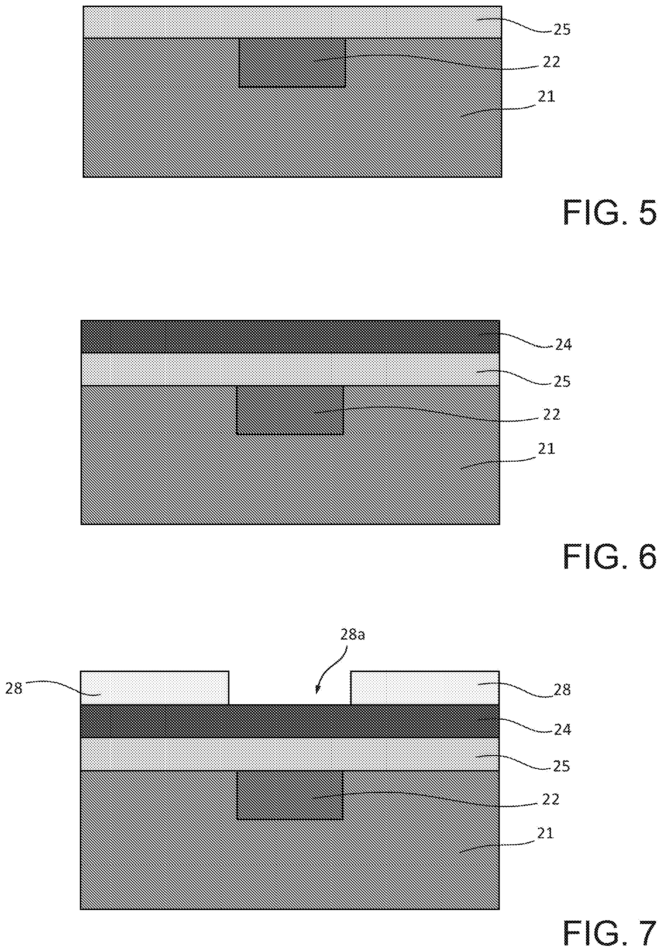

Figure 5 shows a first ILD insulating layer 25 made from Tetraethyl orthosilicate (commonly known as TEOS) and/or Borophosphosilicate glass (commonly known as BPSG) and deposited (for instance by Chemical Vapor Deposition (CVD)) on the semiconductor substrate 21.

Figure 6 shows a first metal layer 24 (for example, made up of a stack of three metal materials, such as Ti, TiN and AlCu) deposited (for instance by CVD or Physical Vapor Deposition (PVD)) on the first ILD insulating layer 25.

Figure 7 shows a photoresist mask 28 patterned by a photoligraphic process to expose (through a window 28a) a portion of the first metal layer 24 (which is above the photodetector active area 22) to a subsequent etch process intended to remove said exposed portion in order to realize the optical path 26.

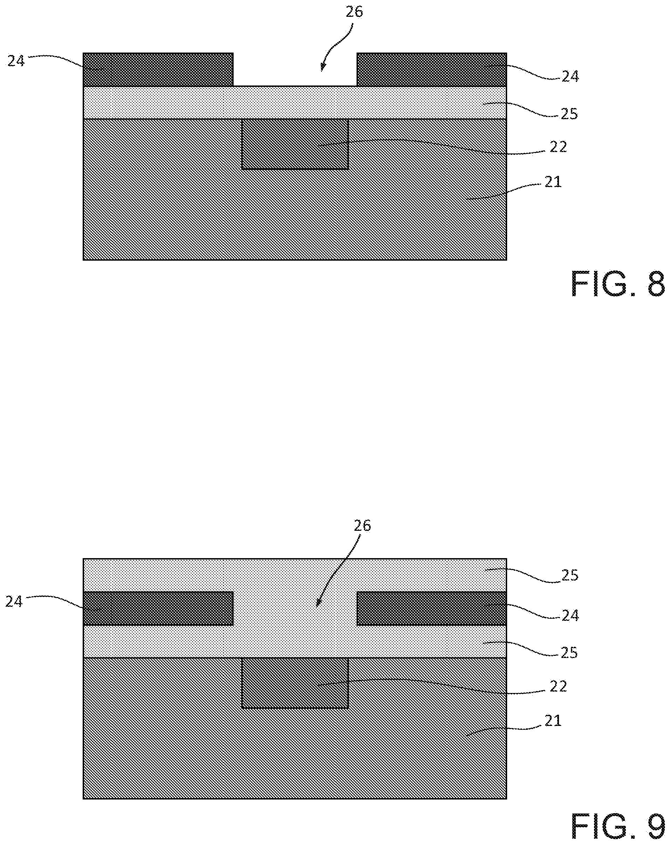

Figure 8 shows the first metal layer 24 patterned after the etch process so as to have the optical path 26 above the photodetector active area 22. For example, a dry etching stopping at the first ILD insulating layer 25 below said first metal layer 24 can be conveniently used.

Figure 9 shows a second ILD insulating layer 25 deposited (for instance a SiO₂ film deposited by low-pressure CVD (LPCVD)) on the first metal layer 24 and filling the optical path 26. In detail, Figure 9 shows the second ILD insulating layer 25 after a planarization process based on Chemical Mechanical Polishing (CMP).

Thence, the multilayer structure 23 can be manufactured by repeating the above manufacturing steps (in particular, from the step shown in Figure 6 up to the step shown in Figure 9).

The above manufacturing process is intended to be used with metal layers based on an aluminum-copper (AlCu) alloy. Otherwise, if metal layers based on copper are used, a manufacturing process based on damascene scheme is conveniently used, wherein a groove pattern is created on the ILD insulating layers 25 and then filled with copper by an electroplating process, followed by a CMP process.

The angular response of the photocell 2 can be tuned by properly adjusting the size (in particular, width and height) of the optical path 26, the size of the photodetector active area 22 (in particular the planar size of the photodetector active area 22 on the top planar surface of the semiconductor substrate 21 where said photodetector active area 22 is exposed), and the relative position of said photodetector active area 22 with respect to the optical path 26 (for example, as shown in Figure 2, the width W1 and the height H1 of the aperture 26 and the width W2 of the photodetector active area 22).

An optical sensor according to the first preferred embodiment of the present invention includes an array of photocells as the one shown in Figure 2 (in particular the structure shown in Figure 2 is replicated for each single photocell in the array). In detail, all the photocells 2 of the array are connected in parallel to provide an overall output electrical signal related to (in particular, indicative of) incident light impinging on all the photodetector active areas 22 (in fact, the overall output electrical signal of the optical sensor is given by the sum of the output electrical signals of all the photocells 2).

Moreover, all the optical paths 26 are parallel to (i.e., the axes d of all the optical paths 26 in the optical sensor are parallel to) one and the same given direction, thereby causing all the photodetector active areas 22 to be reached by incident light with incident direction parallel to said given direction. Conveniently, the optical paths 26 are perpendicular to (i.e., the axes d of the optical paths 26 in the optical sensor are perpendicular to) the top planar surface of the semiconductor substrate 21 where the photodetector active areas 22 are exposed.

Thence, the optical sensor according to the first preferred embodiment of the present invention includes an array of photocells 2 electrically connected in parallel and with photodetector active areas 22 receiving incident light with incident direction parallel to the given direction, thereby simulating the operation of a single big device by means of a plurality of smaller devices.

Preferably, in order to maximize the light shielding effect, the photodetector active areas 22 have one and the same given planar size on the top planar surface of the semiconductor substrate 21 where said photodetector active areas 22 are exposed; moreover, the optical paths 26 have one and the same given height H1 and one and the same given width W1, wherein both said given height H1 and given width W1 are at least comparable to the size of the photodetector active area 22 (in particular the given planar size of the photodetector active areas 22).

Additionally, again with reference to Figure 2, said given planar size, height H1 and width W1, and relative position of each photodetector active area 22 with respect to the corresponding optical path 26:
- define one and the same given angular range for all the photodetector active areas 22; and
- cause the photodetector active areas 22 to be reached only by incident light with incident direction *dᵢ*

- parallel to the axis *d* (or, more in general, parallel to said given direction), or
- defining, with respect to said axis *d* (or, more in general, with respect to said given direction), an incidence angle α falling within said given angular range.

In this connection, it is worth noting that, since the given height H1 of the optical paths 26 depends on the given planar size of the photodetector active areas 22 (in particular the former increases as the latter increase), the fact of using an array of smaller photodetector active areas 22 instead of one or more bigger ones allows to make optical sensors with thickness highly reduced. Moreover, light filtering capabilities of smaller optical paths are superior to those ones of bigger optical paths.

Conveniently, the light shielding means include metal layers that are integrated in the multilayer structure (more conveniently in the optical stack) of an array of photocells and that can be conveniently shaped as metal routes and/or metal plates or a combination of them.

It is worth noting that, as is known, an image sensor includes an array of pixels and require to address every single pixel of the array for the operations of integration, reset and read-out. Therefore, at least two metal interconnect routes are necessary for this task.

On the contrary, with the above optical sensor including small photocells in parallel that operate, as a whole, as a bigger single device, there is no need to address rows and columns of the photocell array. Therefore, one of the metal layers (for example, in the form of a metal plate) acting as light shielding means can be used to address all the photocells, thereby avoiding the need for metal interconnect routes. This results in time and cost optimization in the manufacturing of the optical sensor.

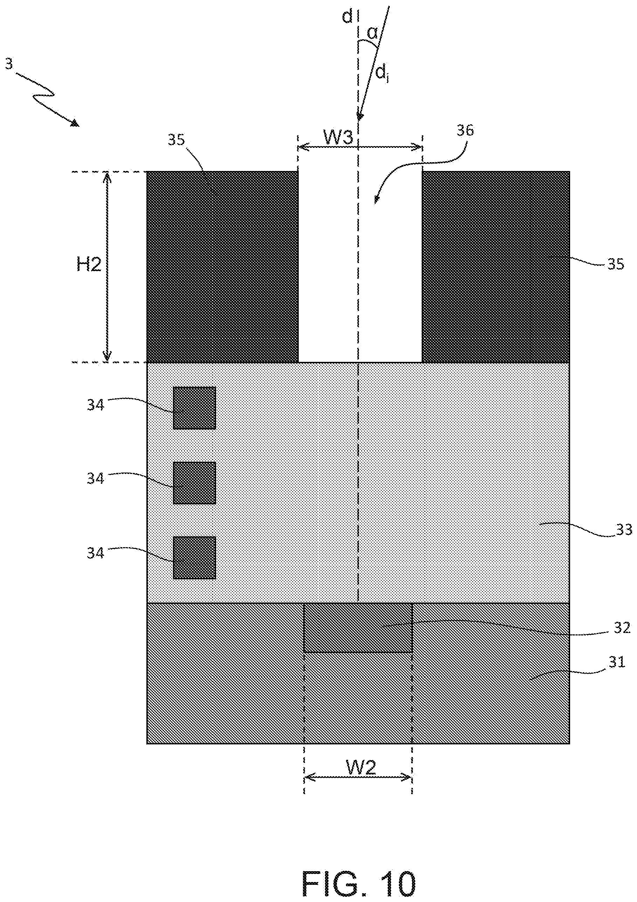

Figure 10 shows an example of photocell (denoted as a whole by 3) with light shielding means according to a second preferred embodiment of the present invention. In particular, Figure 10 is a cross-sectional view of the photocell 3, which includes:
- a semiconductor substrate 31 comprising a photodetector active area 32;
- a multilayer structure 33, formed on the semiconductor substrate 31 and comprising several transparent dielectric layers (transparent to the wavelength of the radiation to be detected) and, at different levels, metal interconnect routes 34;
- an opaque material 35 (opaque to the wavelength of the radiation to be detected), such as an opaque polymer (for example a black photoresist) or a metal, placed on the multilayer structure 33; and
- an optical path 36 formed through the opaque material 35, that is above the photodetector active area 32, extends from the top surface of said opaque material 35 to the upper surface of the multilayer structure 33, and extends symmetrically with respect to, and around, an axis d that passes through the centre of the photodetector active area 32.

The light shielding effect can be tuned by properly adjusting the height H2 and the width W3 of the optical path 36, the width W2 of the photodetector active area 32, and the relative position of the optical path 36 with respect to the photodetector active area 32. In particular, in this way it is possible to define the admissible angular range for the incidence angles α of the incident directions *dᵢ* (with respect to the axis *d*) of the incident light.

An optical sensor according to the second preferred embodiment of the present invention includes an array of photocells as the one shown in Figure 10 (in particular the structure shown in Figure 10 is replicated for each single photocell in the array). In detail, all the photocells 3 of the array are connected in parallel to provide an overall output electrical signal related to (in particular, indicative of) incident light impinging on all the photodetector active areas 32 (in fact, the overall output electrical signal of the optical sensor is given by the sum of the output electrical signals of all the photocells 3).

The advantages of the present invention are clear from the foregoing. In particular, it is important to underline the fact that the light shielding structure according to the present invention allows to limit, in particular to narrow, the angular response of an optical sensor, thereby minimizing the technical problem of wearable biometric monitoring devices due to side light interference.

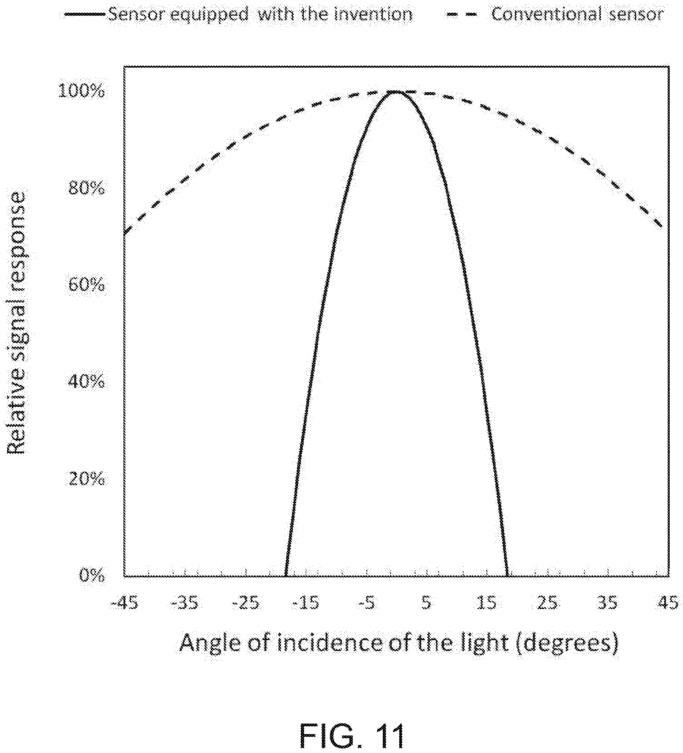

In this respect, Figure 11 shows a comparison between relative signal responses (with respect to angle of incidence of the light) of a conventional optical sensor and an optical sensor according to the present invention, respectively. In particular, in Figure 11 each relative signal response represents the signal output normalized to its maximum value. As it is clearly shown in Figure 11, the optical sensor according to the present invention is characterized by a narrower angular signal response.

Finally, it is worth noting again that US 2007/0290284 A1 discloses a photosensitive device for sensing an illumination direction of radiation incident on the device for adjusting or compensating for the alignment of components of an optical sensing arrangement used in a metrology instrument (e.g., an optical encoder system) or other optical system. In other words, the device according to US 2007/0290284 A1, differently from the present invention, exploits non-parallel optical paths (each coupled with a respective photodetector active area) and an array of photocells not connected in parallel, in order to detect the illumination direction of incident light for metrology purposes. Therefore, the device according to US 2007/0290284 A1 is completely different (as for features and purposes) with respect to the present invention and, thence, does not have the respective technical advantages.

Moreover, it is worth noting also that the solution to the side light problem of wearable biometric monitoring devices proposed in US 2014/0276119 A1 is limited to the use of light-transmissive structures (in particular, structures created through an In-Mould-Labeling (IML) film formed over the photo detector and the light source(s)), wherein said light-transmissive structures may include a mask consisting of an opaque material that limits the aperture of one, some, or all of the light source(s) and/or detector. Therefore, US 2014/0276119 A1 does not anticipate the features of the present invention and, thence, does not have the respective technical advantages.

In conclusion, it is clear that numerous modifications and variants can be made to the present invention, all falling within the scope of the invention, as defined in the appended claims. For example, a combination of the two embodiments previously described and illustrated in Figures 2 and 10 can be also envisaged.

## Claims

1. An optical sensor based on CMOS technology and comprising:
• a semiconductor substrate (21,31);
• an array of photocells (2,3), each of which
- includes a respective photodetector active area (11,12,22,32) that is formed in the semiconductor substrate (21,31) and is exposed on a given planar surface (13) of said semiconductor substrate (21,31), and
- is designed to provide a respective output electrical signal related to incident light impinging on the respective photodetector active area (11,12,22,32);
• a multilayer structure (23,33), that includes metal and dielectric layers and is formed on the given planar surface (13) of the semiconductor substrate (21,31); and
• light shielding means (14,15,24,35), that are formed in or on the multilayer structure (23,33) and are made of one or more materials reflecting and/or absorbing incident light impinging on said light shielding means (14,15,24,35);
wherein each photodetector active area (11,12,22,32) is associated with a corresponding optical path (26,36) extending through the light shielding means (14,15,24,35) and directed towards said photodetector active area (11,12,22,32) to allow incident light with incident direction falling within a given direction range to reach said photodetector active area (11,12,22,32);
**characterized in that**:
• all the photocells (2,3) are connected in parallel to provide an overall output electrical signal related to incident light impinging on all the photodetector active areas (11,12,22,32); and
• all the optical paths (26,36) are parallel to a given direction thereby causing all the photodetector active areas (11,12,22,32) to be reached by incident light with incident direction parallel to said given direction.

2. The optical sensor of claim 1, wherein the optical paths (26,36) are perpendicular to the given planar surface (13) of the semiconductor substrate (21,31).

3. The optical sensor according to claim 1 or 2, wherein each optical path (26,36) is defined by one or more respective coaxial apertures (16,17) formed in the light shielding means (14,15,24,35) and centered on a respective axis parallel to said given direction.

4. The optical sensor according to any claim 1-3, wherein:
• the photodetector active areas (11,12,22,32) have one and the same given planar size on the given planar surface (13) of the semiconductor substrate (21,31);
• the optical paths (26,36) have one and the same given height and one and the same given width, said given height and width being related to said given planar size; and
• said given planar size, height and width, and relative position of each photodetector active area (11,12,22,32) with respect to the corresponding optical path (26, 36)
- define one and the same given angular range for all the photodetector active areas (11,12,22,32), and
- cause the photodetector active areas (11,12,22,32) to be reached only by incident light with incident direction
- parallel to said given direction, or
- defining, with respect to said given direction, an incidence angle falling within said given angular range.

5. The optical sensor according to any preceding claim, wherein the light shielding means (14,15,24,35) are made of one or more metal materials.

6. The optical sensor according to any preceding claim, wherein the light shielding means (14,15,24,35) are formed in one or more metal layers (24) and/or metal interconnects in the multilayer structure (23,33).

7. The optical sensor according to any claim 1-4, wherein the light shielding means (14,15,24,35) are made of an opaque polymer.

8. The optical sensor according to any claim 1-4, wherein the light shielding means (14,15,24,35) are made of a black photoresist.

9. The optical sensor according to claim 7 or 8, wherein the light shielding means (14,15,24,35) are formed on the multilayer structure (23,33).

10. Wearable biometric parameter monitoring device including the optical sensor as claimed in any preceding claim.

## Patentansprüche

1. Optischer Sensor, der auf CMOS-Technologie basiert und umfasst:
• ein Halbleitersubstrat (21, 31);
• ein Array von Fotozellen (2,3), von denen jede
- einen jeweiligen aktiven Fotodetektorbereich (11, 12, 22, 32) enthält, der in dem Halbleitersubstrat (21, 31) ausgebildet ist und auf einer gegebenen planaren Oberfläche (13) des Halbleitersubstrats (21, 31) freigelegt ist, und
- ausgelegt ist, ein jeweiliges elektrisches Ausgangssignal bereitzustellen, das sich auf einfallendes Licht bezieht, das auf den jeweiligen aktiven Fotodetektorbereich (11, 12, 22, 32) auftrifft;
• eine Mehrschichtstruktur (23, 33), die Metallschichten und dielektrische Schichten enthält und auf der gegebenen planaren Oberfläche (13) des Halbleitersubstrats (21, 31) ausgebildet ist; und
• Lichtabschirmmittel (14, 15, 24, 35), die in oder auf der Mehrschichtstruktur (23, 33) ausgebildet sind und aus einem oder mehreren Materialien bestehen, die auf das Lichtabschirmmittel (14, 15, 24, 35) auftreffendes Licht reflektieren und/oder absorbieren;
wobei jedem aktiven Fotodetektorbereich (11, 12, 22, 32) ein entsprechender optischer Pfad (26, 36) zugeordnet ist, der sich durch die Lichtabschirmmittel (14, 15, 24, 35) erstreckt und auf den aktiven Fotodetektorbereich (11, 12, 22, 32) gerichtet ist, damit einfallendes Licht mit einer Einfallsrichtung, die in einen vorgegebenen Richtungsbereich fällt, den aktiven Fotodetektorbereich (11, 12, 22, 32) erreichen kann;
**dadurch gekennzeichnet, dass**:
• alle Fotozellen (2, 3) parallel geschaltet sind, um ein elektrisches Gesamtsignal zu liefern, das sich auf einfallendes Licht bezieht, das auf alle aktiven Fotodetektorbereiche (11, 12, 22, 32) auftrifft; und
• alle optischen Pfade (26, 36) parallel zu einer gegebenen Richtung sind, wodurch bewirkt wird, dass alle aktiven Fotodetektorbereiche (11, 12, 22, 32) durch einfallendes Licht mit einer zur gegebenen Richtung parallelen Einfallsrichtung erreicht werden.

2. Optischer Sensor nach Anspruch 1, wobei die optischen Pfade (26, 36) senkrecht zu der gegebenen planaren Oberfläche (13) des Halbleitersubstrats (21, 31) sind.

3. Optischer Sensor nach Anspruch 1 oder 2, wobei jeder optische Pfad (26, 36) durch eine oder mehrere jeweilige koaxiale Öffnungen (16, 17) definiert ist, die in den Lichtabschirmmitteln (14, 15, 24, 35) ausgebildet sind und auf einer jeweiligen Achse parallel zu der gegebenen Richtung sind.

4. Optischer Sensor nach einem der Ansprüche 1-3, wobei:
• die aktiven Fotodetektorbereiche (11, 12, 22, 32) auf der gegebenen planaren Oberfläche (13) des Halbleitersubstrats (21, 31) ein und dieselbe gegebene planare Größe aufweisen;
• die optischen Pfade (26, 36) ein und dieselbe gegebene Höhe und ein und dieselbe gegebene Breite aufweisen, wobei die gegebene Höhe und Breite mit der gegebenen planaren Größe in Beziehung stehen; und
• die gegebene planare Größe, Höhe und Breite und relative Position jedes aktiven Fotodetektorbereichs (11, 12, 22, 32) in Bezug auf den entsprechenden optischen Pfad (26, 36)
- ein und denselben Winkelbereich für alle aktiven Fotodetektorbereiche (11, 12, 22, 32) definieren, und
- bewirken, dass die aktiven Fotodetektorbereiche (11, 12, 22, 32) nur durch einfallendes Licht mit Einfallsrichtung
- parallel zu der gegebenen Richtung oder
- mit Einfallsrichtung erreicht werden, die in Bezug auf die gegebene Richtung einen Einfallswinkel definiert, in den gegebenen Winkelbereich fällt.

5. Optischer Sensor nach einem der vorhergehenden Ansprüche, wobei die Lichtabschirmmittel (14, 15, 24, 35) aus einem oder mehreren Metallmaterialien bestehen.

6. Optischer Sensor nach einem der vorhergehenden Ansprüche, wobei die Lichtabschirmmittel (14, 15, 24, 35) in einer oder mehreren Metallschichten (24) und/oder Metallverbindungen in der Mehrschichtstruktur (23, 33) ausgebildet sind.

7. Optischer Sensor nach einem der Ansprüche 1-4, wobei die Lichtabschirmmittel (14, 15, 24, 35) aus einem opaken Polymer bestehen.

8. Optischer Sensor nach einem der Ansprüche 1-4, wobei die Lichtabschirmmittel (14, 15, 24, 35) aus einem schwarzen Fotolack bestehen.

9. Optischer Sensor nach Anspruch 7 oder 8, wobei die Lichtabschirmmittel (14, 15, 24, 35) auf der Mehrschichtstruktur (23, 33) ausgebildet sind.

10. Tragbare biometrische Parameterüberwachungsvorrichtung mit dem optischen Sensor nach einem der vorhergehenden Ansprüche.

## Revendications

1. Capteur optique basé sur la technologie CMOS et comprenant :
- un substrat à semi-conducteur (21, 31) ;
- un réseau de photocellules (2, 3), dont chacune
-- comporte une zone active de photodétecteur (11, 12, 22, 32) respective qui est formée dans le substrat à semi-conducteur (21, 31) et est exposée sur une surface plane donnée (13) dudit substrat à semi-conducteur (21, 31), et
-- est conçue pour fournir un signal électrique de sortie respectif lié à une lumière incidente arrivant sur la zone active de photodétecteur (11, 12, 22, 32) respective ;
- une structure multicouche (23, 33), qui comporte des couches en métal et des couches diélectriques et est formée sur la surface plane donnée (13) du substrat à semi-conducteur (21, 31) ; et
- des moyens pare-lumière (14, 15, 24, 35), qui sont formés dans ou sur la structure multicouche (23, 33) et sont réalisés en un ou plusieurs matériaux réfléchissants et/ou absorbants une lumière incidente arrivant sur lesdits moyens pare-lumière (14, 15, 24, 35) ;
dans lequel chaque zone active de photodétecteur (11, 12, 22, 32) est associée à un chemin optique (26, 36) correspondant s'étendant à travers les moyens pare-lumière (14, 15, 24, 35) et dirigé vers ladite zone active de photodétecteur (11, 12, 22, 32) pour permettre à une lumière incidente avec une direction incidente s'inscrivant dans une plage de direction donnée d'atteindre ladite zone active de photodétecteur (11, 12, 22, 32) ;
**caractérisé en ce que** :
- toutes les photocellules (2, 3) sont connectées en parallèle pour fournir un signal électrique de sortie global lié à une lumière incidente arrivant sur toutes les zones actives de photodétecteur (11, 12, 22, 32) ; et
- tous les chemins optiques (26, 36) sont parallèles à une direction donnée amenant ainsi toutes les zones actives de photodétecteur (11, 12, 22, 32) à être atteintes par une lumière incidente avec une direction incidente parallèle à ladite direction donnée.

2. Capteur optique selon la revendication 1, dans lequel les chemins optiques (26, 36) sont perpendiculaires à la surface plane donnée (13) du substrat à semi-conducteur (21, 31).

3. Capteur optique selon la revendication 1 ou 2, dans lequel chaque chemin optique (26, 36) est défini par une ou plusieurs ouvertures coaxiales (16, 17) respectives formées dans les moyens pare-lumière (14, 15, 24, 35) et centrées sur un axe respectif parallèle à ladite direction donnée.

4. Capteur optique selon l'une quelconque des revendications 1 à 3, dans lequel :
- les zones actives de photodétecteur (11, 12, 22, 32) ont une seule et même taille plane donnée sur la surface plane donnée (13) du substrat à semi-conducteur (21, 31) ;
- les chemins optiques (26, 36) ont une seule et même hauteur donnée et une seule et même largeur donnée, lesdites hauteur et largeur données étant liées à ladite taille plane donnée ; et
- lesdites taille plane, hauteur et largeur données, et la position relative de chaque zone active de photodétecteur (11, 12, 22, 32) par rapport au chemin optique (26, 36) correspondant
-- définissent une seule et même plage angulaire donnée pour toutes les zones actives de photodétecteur (11, 12, 22, 32), et
-- amènent les zones actives de photodétecteur (11, 12, 22, 32) à être atteintes uniquement par une lumière incidente avec une direction incidente
-- parallèle à ladite direction donnée, ou
-- définissant, par rapport à ladite direction donnée, un angle d'incidence s'inscrivant dans ladite plage angulaire donnée.

5. Capteur optique selon une quelconque revendication précédente, dans lequel les moyens pare-lumière (14, 15, 24, 35) sont réalisés en un ou plusieurs matériaux en métal.

6. Capteur optique selon une quelconque revendication précédente, dans lequel les moyens pare-lumière (14, 15, 24, 35) sont formés en une ou plusieurs couches en métal (24) et/ou interconnexions en métal dans la structure multicouche (23, 33).

7. Capteur optique selon l'une quelconque des revendications 1 à 4, dans lequel les moyens pare-lumière (14, 15, 24, 35) sont réalisés en un polymère opaque.

8. Capteur optique selon l'une quelconque des revendications 1 à 4, dans lequel les moyens pare-lumière (14, 15, 24, 35) sont réalisés en une photorésine noire.

9. Capteur optique selon la revendication 7 ou 8, dans lequel les moyens pare-lumière (14, 15, 24, 35) sont formés sur la structure multicouche (23, 33).

10. Dispositif de surveillance de paramètre biométrique portable comportant le capteur optique tel que revendiqué à une quelconque revendication précédente.
